# EUROPEAN PATENT SPECIFICATION

(11) **EP 3 833 186 B1**
(45) Date of publication and mention of the grant of the patent: **08.07.2026**
(21) Application number: 19779106.4
(22) Date of filing: 05.08.2019
(51) Int. Cl.: A01N 33/04, A01N 43/40, A01N 47/44, A01N 25/30, A01N 25/24, A01N 47/12, A01P 1/00

(54) **THERAPEUTIC COMPOSITION AND RELATED METHODS**
THERAPEUTISCHE ZUSAMMENSETZUNGEN UND ZUGEHÖRIGE VERFAHREN
COMPOSITION THÉRAPEUTIQUE ET PROCÉDÉS ASSOCIÉS

(30) Priority: 08.08.2018 US 201862715991 P
(43) Date of publication of application: 16.06.2021
(73) Proprietor: Solventum Intellectual Properties Company, Eagan, MN 55121 (US)
(72) Inventor: KOHLER RIEDI, Petra L., Saint Paul, Minnesota 55133-3427 (US); BARAN, Jimmie R. Jr., Saint Paul, Minnesota 55133-3427 (US); YANG, Jie, Saint Paul, Minnesota 55133-3427 (US); PARTHASARATHY, Ranjani V., Saint Paul, Minnesota 55133-3427 (US)
(74) Representative: Mathys & Squire
(86) International application number: PCT/IB2019/056648
(87) International publication number: WO 2020/031065

(56) References cited:
- WO-A1-2017/205230
- WO-A1-2017/205230
- US-A1- 2002 068 014
- US-A1- 2002 068 014
- M. ADDY ET AL: "Studies on the effect of toothpaste rinses on plaque regrowth. (I). Influence of surfactants on chlorhexidine efficacy.", J CLIN PERIODONTOL., vol. 16, no. 6, 1 July 1989 (1989-07-01), pages 380 - 384, XP055651204, DOI: : 10.1111/j.1600-051x.1989.tb00008.x
- M. ADDY ET AL: "Studies on the effect of toothpaste rinses on plaque regrowth. (I). Influence of surfactants on chlorhexidine efficacy.", J CLIN PERIODONTOL., vol. 16, no. 6, 1 July 1989 (1989-07-01), pages 380 - 384, XP055651204, DOI: : 10.1111/j.1600-051x.1989.tb00008.x

## Description

### BACKGROUND

WO 2017/205230 A1 describes dental pastes including:
(i) a compound of the following Formula: 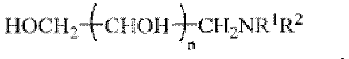
(ii) a compound of the following Formula 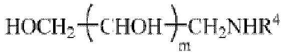 or a pharmaceutically acceptable salt thereof: and (iii) a dental abrasive.

M. Addy et al report in J Clin Periodontol, . 1989 Jul;16(6):380-4, "Studies on the effect of toothpaste rinses on plaque regrowth. (I). Influence of surfactants on chlorhexidine efficacy".

US 2002/068014A1 describes to antibacterial agents, more particularly salicylanilide substituted compositions, preferably monosubstituted salicylanilide compositions, most preferably monohalogenated salicylanilide compositions, useful in antibacterial compositions, bacteria-reducing systems, antibacterial products and bacteria-reducing methods.

The ability to fight bacteria has been a longstanding and valued feature in medical care products. To date, these benefits have been attained via antimicrobial agents. The use of antimicrobial agents such as antibiotics plays an important part in current medical therapy. For decades medicine has relied primarily upon antibiotics to fight systemic as well as topical infections. However, due to safety and environmental concerns with antimicrobial agents, there is still a need for a better antimicrobial compositions.

### SUMMARY

Thus, in one aspect, the present disclosure provides a composition as set out in claim 1. The composition comprises: octanoyl-N-methylglucamide (MEGA-8) (i.e. a compound of Formula (I): HOCH₂-(--CHOHₙ)n-CH2NR¹R² (I) where n is 4, R¹ is CH₃ and R² is C(O)R³, where R³ is C₇H₁₅); wherein a concentration of the compound of octanoyl-N-methyl-glucamide (MEGA-8) is more than 170% of its critical micelle concentration; and an antimicrobial agent selected from the group consisting of a biguanide antimicrobial agent and a polymeric, cationic, non-micelle forming antimicrobial material; wherein a concentration of the antimicrobial agent is no more than 5 wt%.

In another aspect, the present disclosure provides a composition as set out in claim 5. The composition comprises: octanoyl-N-methylglucamide (MEGA-8); wherein a concentration of octanoyl-N-methylglucamide (MEGA-8) more than 3.2 wt%; and an antimicrobial agent selected from the group consisting of a biguanide antimicrobial agent and a polymeric, cationic, non-micelle forming antimicrobial material; wherein a concentration of the antimicrobial agent is no more than 5 wt%.

In methods of use, compositions of the present disclosure may be applied to a surface of a subject or a medical device.

Various aspects and advantages of exemplary embodiments of the present disclosure have been summarized. The above Summary is not intended to describe each illustrated embodiment or every implementation of the present disclosure. Further features and advantages are disclosed in the embodiments that follow. The Drawings and the Detailed Description that follow more particularly exemplify certain embodiments using the principles disclosed herein.

### DEFINITIONS

For the following defined terms, these definitions shall be applied for the entire Specification, including the claims, unless a different definition is provided in the claims or elsewhere in the Specification based upon a specific reference to a modification of a term used in the following definitions:
The terms "about" or "approximately" with reference to a numerical value or a shape means +/- five percent of the numerical value or property or characteristic, but also expressly includes any narrow range within the +/- five percent of the numerical value or property or characteristic as well as the exact numerical value. For example, a temperature of "about" 100°C refers to a temperature from 95°C to 105°C, but also expressly includes any narrower range of temperature or even a single temperature within that range, including, for example, a temperature of exactly 100°C. For example, a viscosity of "about" 1 Pa-sec refers to a viscosity from 0.95 to 1.05 Pa-sec, but also expressly includes a viscosity of exactly 1 Pa-sec. Similarly, a perimeter that is "substantially square" is intended to describe a geometric shape having four lateral edges in which each lateral edge has a length which is from 95% to 105% of the length of any other lateral edge, but which also includes a geometric shape in which each lateral edge has exactly the same length.

The term "substantially" with reference to a property or characteristic means that the property or characteristic is exhibited to a greater extent than the opposite of that property or characteristic is exhibited. For example, a substrate that is "substantially" transparent refers to a substrate that transmits more radiation (e.g. visible light) than it fails to transmit (e.g. absorbs and reflects). Thus, a substrate that transmits more than 50% of the visible light incident upon its surface is substantially transparent, but a substrate that transmits 50% or less of the visible light incident upon its surface is not substantially transparent.

The term "CMC (critical micelle concentration)" with reference to a concentration of a surfactant in a liquid (eg water) is when the air/liquid interface is completely saturated with surfactant molecules and further addition of surfactant molecules will result in the formation of micelles in the bulk liquid (eg water). CMC used in the present disclosure refers to the reported CMC of a surfactant alone in water at 25°C.

The terms "a", "an", and "the" include plural referents unless the content clearly dictates otherwise. Thus, for example, reference to a material containing "a compound" includes a mixture of two or more compounds.

### DETAILED DESCRIPTION

Before any embodiments of the present disclosure are explained in detail, it is understood that the claimed invention is not limited in its application to the details of use, construction, and the arrangement of components set forth in the following description. The invention is capable of other embodiments and of being practiced or of being carried out in various ways that will become apparent to a person of ordinary skill in the art upon reading the present disclosure. Also, it is understood that the phraseology and terminology used herein is for the purpose of description and should not be regarded as limiting. The use of "including," "comprising," or "having" and variations thereof herein is meant to encompass the items listed thereafter. It is understood that other embodiments may be utilized and structural or logical changes may be made without departing from the scope of the present invention as set out in the appended claims.

As used in this Specification, the recitation of numerical ranges by endpoints includes all numbers subsumed within that range (e.g. 1 to 5 includes 1, 1.5, 2, 2.75, 3, 3.8, 4, and 5, and the like).

Unless otherwise indicated, all numbers expressing quantities or ingredients, measurement of properties and so forth used in the Specification and embodiments are to be understood as being modified in all instances by the term "about." Accordingly, unless indicated to the contrary, the numerical parameters set forth in the foregoing specification and attached listing of embodiments can vary depending upon the desired properties sought to be obtained by those skilled in the art utilizing the teachings of the present disclosure. At the very least, and not as an attempt to limit the application of the doctrine of equivalents to the scope of the claimed embodiments, each numerical parameter should at least be construed in light of the number of reported significant digits and by applying ordinary rounding techniques.

The literature reports that non-ionic surfactants above their reported CMC do not enhance the anti-microbial performance of quaternary ammonium salts, presumably because the non-ionic surfactants form mixed micelles with these materials (Disinfection, Sterilization, and Preservation, edited by S.S. Block, 2nd edition, Lea & Febiger, Philadelphia, 1977). Similarly, it is widely reported that non-ionic surfactants above their reported CMC do not enhance the anti-microbial performance of biguanide antimicrobial agents at high surfactant concentrations, presumably because there is an interaction between surfactant micelles and the biguanide antimicrobial agents (eg. chlorhexidine gluconate) (reviewed in Block, 1977). Therefore, while many non-ionic surfactants enhance the activity of antimicrobial agents for killing microorganisms at sub-CMC (critical micelle concentration) concentrations, the enhancement is diminished once the CMC is exceeded or enhancement does not further improve. The current disclosure provides composition comprising a non-ionic surfactant which enhance the activity of antimicrobial agents at concentrations above the CMC further over the enhancement observed below the CMC. Surprisingly, the surfactant MEGA-8 (octanoyl-N-methylglucamide) is synergistic with a small subset of antimicrobial agents at unexpectedly high concentrations of the MEGA-8 surfactant. For example, the MEGA-8 surfactant can provide a synergistic enhancement of antimicrobial activity for biguanide antimicrobial agents and polymeric, cationic, non-micelle forming antimicrobial materials. This discovery allows for the formulation of compositions with reduced concentrations of antimicrobial agents, and for the formulation of compositions where antimicrobial activity is needed in combination with the cleaning ability of high surfactant concentrations.

In one aspect, the composition of the present disclosure comprises:
octanoyl-N-methylglucamide (MEGA-8), wherein a concentration of octanoyl-N-methylglucamide (MEGA-8) is more than 170% of its critical micelle concentration (CMC) in water at 25°C; and
an antimicrobial agent selected from the group consisting of a biguanide antimicrobial agent and a polymeric, cationic, non-micelle forming antimicrobial material, wherein a concentration of the antimicrobial agent is no more than 5 wt%.

In another aspect, the present disclosure provides a composition comprising: octanoyl-N-methylglucamide (MEGA-8); herein a concentration of octanoyl-N-methylglucamide (MEGA-8) more than 3.2 wt%; and an antimicrobial agent selected from the group consisting of a biguanide antimicrobial agent and a polymeric, cationic, non-micelle forming antimicrobial material; wherein a concentration of the antimicrobial agent is no more than 5 wt%.

The biguanide antimicrobial agent can include those decribed in US 2016/0213001 (Parthasarathy and Scholz et al). In some embodiments, the biguanide antimicrobial agent can include chlorhexidine (which is the common name for the antiseptic 1, 1'-hexamethylenebis-[5-(4-chlorophenyl) -biguanide] widely used in the form of its salts (such as the acetate, hydrochloride, and gluconate salts) in the cosmetic and pharmaceutical fields and also in cleaning preparations). Other salts of chlorhexidine include formate, lactate, isethionate, succinamate, glutamate, mono-diglycollate, dimethanesulfonate, di-isobutyrate, glucoheptonate. Preferably, the chlorhexidine salts are gluconate and acetate, the most preferred being chlorhexidine digluconate.

Additional examples of antimicrobial biguanide agents, which can be utilized in the present invention can include N¹-(4 chlorobenzyl)-N⁵-(2,4-dichlorobenzyl)-biguanide; p-chlorophenyl biguanide; 4-chlorobenzhydryl biguanide; N-3-lauroxypropyl-N⁵-p-chlorobenzyl biguanide; N¹-p chlorophenyl-N⁵-lauryl biguanide; Olanedine (olanexidine gluconate) and the non-toxic addition salts thereof, especially gluconates and acetates. Polymeric biguanide antimicrobial agents such as polyhexamethylene biguanide and its salts can also be used.

The polymeric, cationic, non-micelle forming antimicrobial material can inlcude those decribed in US 8,338,491 (Asmus and Hobbs). In some embodiments, the polymeric, cationic, non-micelle forming antimicrobial material can inlcude polymers having quaternary amine groups. These polymers typically have at least one alkyl or aralkyl chain of at least 6 carbon atoms and preferably at least 8 carbon atoms. The polymers may be linar, branched, hyperbranched, or dendrimers. Polymeric, cationic, non-micelle forming antimicrobial materials can include polyethyleneimine (PEI), polymeric quaternary ammonium salts, or chitosan.

In some embodiments, the composition can further comprise a glucamine compound. Glucamine compound can be any suitable glucamine compound, for example, amino sugar alcohols and derivatives, including D-glucamine, N-methyl-D-glucamine, N-Ethyl-D-Glucamine, N-Propyl-Glucamine, N-Butyl-D-Glucamine, N-octyl-D-glucamine, and combinations thereof.

In some embodiments, the composition can further comprise a glucosamine compound. Glucosamine compound can be any suitable glucamine compound, for example, D-Glucosamine Hydrochloride, D-Glucosamine Sulfate, D-Glucosamine Phosphate, D-Glucosamine gluconate and combinations thereof.

In some embodiments, the composition can further comprise a pharmaceutically acceptable carrier or solvent. The pharmaceutically acceptable carrier may comprise a liquid, a solid, or a gel. In some embodiments, the carrier may be a liquid at about room temperature. In other embodiments, the carrier may be a solid at about room temperature. In some embodiments, the carrier may be a liquid at about the temperature of the oral cavity of a human, i.e., at about 37° C. In other embodiments, the carrier may be a solid at about the temperature of the oral cavity of a human. Exemplary liquid carriers include water, alcohol, glycerol, polyethylene glycol, triethanolamine, methoxypropanol, isopropanol, ethyl acetate, polypropylene glycol and a combination thereof. Exemplary solid carriers include polymers such as natural rubber, butyl rubber, poly(isobutylene), elastomers, styrene-butadiene rubber, polysaccharides, and waxes (e.g., beeswax).

Each non-carrier component of the composition may independently be dissolved, dispersed, suspended, or emulsified in the carrier. In some embodiments, at least one component of the composition is dissolved in the carrier. In some embodiments, at least one component of the composition is dispersed in the carrier. In some embodiments, at least one component of the composition is suspended in the carrier. In some embodiments, at least one component of the composition is emulsified in the carrier.

In some embodiments, the composition can further comprise a binder. The binder may provide a reservoir of a composition comprising a compound of MEGA-8. The composition may be released from the binder. The binder may hold a composition comprising a compound of MEGA-8. In some embodiments, the binder can comprise an acrylic polymer. Suitable acrylic polymers include polymers and copolymers of lower alkyl esters of acrylic or methacrylic acids. In some embodiments, the binder can comprise natural polysaccharides and derivatives selected from murein, pectins, hyaluronate, chondroitin-4-sulfate, dermatan sulfate, keratan sulfate, heparin, heparan sulfate, hydroxyethyl cellulose, hydroxypropyl cellulose, guar gum, and combinations thereof.

In some embodiments, the composition can further comprise sugar-alcohol humectant including xylitol, sorbitol, mannitol and erythritol.

In some embodiments, the composition can be in a form selected from the group consisting of a solution, a dispersion, a suspension, an emulsion, a solid, a paste, a foam, a gel, a spray, a wipe, a mitt and a brush. Any component of the composition may be dissolved, dispersed, suspended, or emulsified in any other component of the composition. In some embodiments, the components are mutually soluble (i.e., miscible with each other).

In some embodiments, the composition may comprise a concentration of MEGA-8 more than 180% of its critical micelle concentration (CMC), more than 190% of its CMC, more than 200% of its CMC, more than 250% of its CMC, more than 300% of its CMC, more than 400% of its CMC, or more than 500% of its CMC. CMC used in the current disclosure refers to the CMC of the compound of Mega-8 alone in water at 25°C.

In some embodiments, the composition may comprise a concentration of the compound of MEGA-8 more than 4 wt%, more than 6 wt%, more than 10 wt%, more than 12 wt%, more than 16 wt%, more than 20 wt%, more than 30 wt%, more than 40 wt%

In certain embodiments, the composition may provide a concentration of a compound of MEGA-8, up to about the solubility limit of the compound, in a medium such as, for example, water, culture broth, or saliva. It is recognized that the solubility limit may be different in different media. In other embodiments, the composition may comprise a concentration of a compound of MEGA-8, greater than about the solubility limit of the compound, in a binder or in a pharmaceutically acceptable carrier.

In some embodiments, the composition may comprise a concentration of the antimicrobial agent no more than 4 wt%, no more than 3 wt%, or no more than 2 wt%. The compositon of the current disclosure can can provide a synergistic enhancement of antimicrobial activity for biguanide antimicrobial agents and polymeric, cationic, non-micelle forming antimicrobial materials, therefore allowing for the use of lower concentrations of antimicrobial materials in order to achieve microbial kill.

In methods for inhibiting microbial growth, compositions of the present invention may be applied to a surface of a subject or a medical device. The surface can be a surface in the oral cavity of a subject includes, for example, a buccal surface, a gingival surface, a tooth, a dental restoration, and bone. The composition may be applied to the oral cavity of a subject by, for example, immersing, inserting, rinsing, spraying, brushing, swabbing, or combinations thereof. Spraying the composition may provide the composition in the form of, for example, an aerosol or a fine mist. The subject may be a human, or the subject may be a non-human animal. Non-human animals include mammals such as canines and felines.

The following working examples are intended to be illustrative of the present disclosure and not limiting.

### EXAMPLES

### Materials

Chlorhexidine gluconate (CHG) was obtained as a 20% solution in water from MP Biomedicals, Santa Ana, CA.

Nonanoyl-N-methylglucamide (MEGA-9) (CAS No. 85261-19-4) was obtained from the Sigma-Aldrich Corporation, St. Louis, MO.

Octanoyl-N-methylglucamide (MEGA-8) (CAS No. 85316-98-9) was obtained from the Sigma-Aldrich Corporation.

TERGITOL 15-S-7 secondary alcohol ethoxylate surfactant was obtained from the Dow Chemical Company, Midland, MI.

Octenidine hydrochloride was obtained from TCI America, Portland, OR.

Cetylpyridinium chloride (CPC) was obtained from MP Biomedicals.

Polyethyleneimine (PEI) (10,000 molecular weight) was obtained from Polysciences Incorporated, Warrington, PA.

Polyhexamethylene biguanide hydrochloride (PHMB) was obtained as a 20% solution in water (weight/weight) under the trade name COSMOCIL PG from the Lonza Group, Basel, Switzerland. Phosphate buffered saline (PBS) was obtained from Thermo Fisher Scientific Incorporated, Waltham, MA.

Dey/Englay (D/E) neutralizing broth and tryptic soy broth were obtained from the Becton, Dickinson and Company, Franklin Lakes, NJ.

Compositions were prepared by combining surfactants and antimicrobial agents in deionized water. Surfactant concentrations were determined as percentages of the published CMCs in water at 25 °C. Compositions containing MEGA-9 surfactant were prepared based on the reported CMC of 20 mM (Sigma-Aldrich product information). Compositions containing MEGA-8 surfactant were prepared based on the reported CMC of 58 mM (Sigma-Aldrich product information). In Tables 1 and 2, the concentrations (mM) of MEGA-9 and MEGA-8 are provided with the corresponding weight percent (wt%) value and the corresponding value for percent of the CMC concentration.

**Table 1.**

| MEGA-9 Surfactant Conc. (mM) | MEGA-9 Surfactant Conc. in Water (wt%) | MEGA-9 (Percent of the CMC Concentration) |
|---|---|---|
| 2 | 0.0672 | 10 |
| 5 | 0.168 | 25 |
| 15 | 0.504 | 75 |
| 18 | 0.605 | 90 |
| 20 | 0.672 | 100 |
| 22 | 0.739 | 110 |
| 25 | 0.840 | 125 |
| 35 | 1.176 | 175 |
| 38 | 1.277 | 190 |
| 40 | 1.344 | 200 |

**Table 2.**

| MEGA-8 Surfactant Conc. (mM) | MEGA-8 Surfactant Conc. in Water (wt%) | MEGA-8 (Percent of the CMC Concentration) |
|---|---|---|
| 5.8 | 0.186 | 10 |
| 14.5 | 0.465 | 25 |
| 43.5 | 1.396 | 75 |
| 52.2 | 1.676 | 90 |
| 58 | 1.862 | 100 |
| 63.8 | 2.048 | 110 |
| 72.5 | 2.327 | 125 |
| 101.5 | 3.258 | 175 |
| 110.2 | 3.537 | 190 |

Comparative Example Compositions containing TERGITOL 15-S-7 surfactant (comparative surfactant) were prepared based on the reported CMC of 7.57 mM (Dow Chemical product data sheet). In Table 3, concentrations (mM) of TERGITOL 15-S-7 are provided with the corresponding weight percent (wt%) value and the corresponding value for percent of the CMC concentration.

**Table 3.**

| TERGITOL 15-S-7 Surfactant Conc. (mM) | TERGITOL15-S-7 Surfactant Conc. in Water (wt%) | TERGITOL 15-S-7 (Percent of the CMC Concentration) |
|---|---|---|
| 0.757 | 0.00039 | 10 |
| 1.89 | 0.00098 | 25 |
| 5.68 | 0.00292 | 75 |
| 6.81 | 0.00351 | 90 |
| 7.57 | 0.00390 | 100 |
| 8.33 | 0.00429 | 110 |
| 9.46 | 0.00488 | 125 |
| 13.2 | 0.00663 | 170 |
| 14.4 | 0.00741 | 190 |

### Test method for the Microbial Kill Assay

*Staphylococcus aureus* strain 15981 was grown to stationary phase in tryptic soy broth at 37 °C. The culture (4 mL) was centrifuged at 13000 RPM for 5 minutes in an Eppendorf Microcentrifuge (Eppendorf Company, Hamburg, Germany) in order to pellet the cells. The bacteria were then resuspended in 2 mL of sterile, deionized water, centrifuged again, and finally resuspended in 10 mL of sterile, deionized water.

The antimicrobial compositions of the examples (180 microliters each) were added to the wells of a polystyrene, 96-well plate. Each well contained a single antimicrobial composition and each composition was tested in triplicate. Control wells were prepared that contained 180 microliters of PBS instead of an antimicrobial composition. The bacterial suspension (20 microliters) was then added to each well. After an exposure period of either 30, 60 or 120 seconds, a 20 microliter aliquot was removed from each well and transferred to 180 microliters of Dey/Englay (D/E) neutralizing broth. The D/E broth was serially diluted (10-fold) with PBS and 100 microliter aliquots of each dilution were transferred to PETRIFILM Aerobic Count Plates (3M Corporation, Maplewood, MN). The PETRIFILM plates were incubated for 24 hours at 37 °C and surviving colony forming units (CFUs) were counted. The average log(CFU/mL) of surviving bacteria was calculated for each composition. The reduction in colony count relative to the PBS control was calculated by subtracting the average surviving log(CFU/mL) of bacteria exposed to a composition from the average log(CFU/mL) of bacteria exposed to the PBS control solution.

### Reference Examples 1-9 and Comparative Example A

Compositions of Reference Examples 1-9 (not according to the present claimed invention) were prepared by combining varying concentrations of MEGA-9 surfactant with CHG (0.1 wt%) in deionized water at 25 °C. The composition of Comparative Example A was prepared to include CHG (0.1 wt%) without any MEGA-9 surfactant. Each composition was exposed to the *S. aureus* suspension for 60 seconds according to the procedure of the "Microbial Kill Assay' described above. The results for Reference Examples 1-9, Comparative Example A, and the PBS Control Solution are reported in Table 4.

**Table 4.**

| | Percent MEGA-9 Surfactant relative to CMC | CHG Conc. (wt%) | Average Surviving Bacteria log(CFU)/mL | Bacteria Reduction log(CFU)/mL relative to Control |
|---|---|---|---|---|
| Ref-Example 1 | 10 | 0.1 | 5.45 | 3.34 |
| Ref-Example 2 | 25 | 0.1 | 5.51 | 3.28 |
| Ref-Example 3 | 75 | 0.1 | 4.85 | 3.94 |
| Ref-Example 4 | 90 | 0.1 | 4.46 | 4.33 |
| Ref-Example 5 | 100 | 0.1 | 4.68 | 4.11 |
| Ref-Example 6 | 110 | 0.1 | 4.52 | 4.27 |
| Ref-Example 7 | 125 | 0.1 | 4.69 | 4.10 |
| Ref-Example 8 | 175 | 0.1 | 4.84 | 3.95 |
| Ref-Example 9 | 190 | 0.1 | 4.30 | 4.49 |
| Comparative Example A | 0 | 0.1 | 6.16 | 2.62 |
| PBS Control | 0 | 0 | 8.79 | |

### Reference Examples 10-12 and Comparative Example B

Compositions of Reference Examples 10-12 (not according to the present claimed invention) were prepared by combining varying concentrations of MEGA-9 surfactant with polyhexamethylene biguanide hydrochloride (PHMB) (0.05 wt%) in deionized water at 25 °C. The composition of Comparative Example B was prepared to include PHMB (0.05 wt%) without any MEGA-9 surfactant. Each composition was exposed to the *S. aureus* suspension for 30 seconds according to the procedure of the 'Microbial Kill Assay' described above. The results for Reference Examples 10-12, Comparative Example B, and the PBS Control Solution are reported in Table 5.

**Table 5.**

| | Percent MEGA-9 Surfactant relative to CMC | PHMB Conc. (wt%) | Average Surviving Bacteria log(CFU)/mL | Bacteria Reduction log(CFU)/mL relative to Control |
|---|---|---|---|---|
| Ref-Example 10 | 10 | 0.05 | 6.86 | 1.20 |
| Ref-Example 11 | 50 | 0.05 | 5.45 | 2.61 |
| Ref-Example 12 | 200 | 0.05 | 4.27 | 3.79 |
| Comparative Example B | 0 | 0.05 | 6.82 | 1.24 |
| PBS Control | 0 | 0 | 8.06 | |

### Reference Examples 13-20, Example 21 and Comparative Example C

Compositions of Reference Examples 13-20 (not according to the claimed invention) and Example 21 were prepared by combining varying concentrations of MEGA-8 surfactant with CHG (0.1 wt%) in deionized water at 25 °C. The composition of Comparative Example C was prepared to include CHG (0.1 wt%) without any MEGA-8 surfactant. Each composition was exposed to the *S. aureus* suspension for 30 seconds according to the procedure of the "Microbial Kill Assay' described above. The results for Reference Examples 13-20, Example 21, Comparative Example C, and the PBS Control Solution are reported in Table 6.

**Table 6:**

| | Percent MEGA-8 Surfactant relative to CMC | CHG Conc. (wt%) | Average Surviving Bacteria log(CFU)/mL | Bacteria Reduction log(CFU)/mL relative to Control |
|---|---|---|---|---|
| Ref-Example 13 | 10 | 0.1 | 7.60 | 1.34 |
| Ref-Example 14 | 25 | 0.1 | 8.05 | 0.89 |
| Ref-Example 15 | 75 | 0.1 | 8.44 | 0.50 |
| Ref-Example 16 | 90 | 0.1 | 8.45 | 0.49 |
| Ref-Example 17 | 100 | 0.1 | 7.70 | 1.24 |
| Ref-Example 18 | 110 | 0.1 | 7.92 | 1.02 |
| Ref-Example 19 | 125 | 0.1 | 7.91 | 1.03 |
| Ref-Example 20 | 170 | 0.1 | 7.16 | 1.78 |
| Example 21 | 190 | 0.1 | 6.90 | 2.04 |
| Comparative Example C | 0 | 0.1 | 8.35 | 0.59 |
| PBS Control | 0 | 0 | 8.94 | |

### Reference Examples 22-27 and Comparative Example D

Compositions of Reference Examples 22-27 (not according ot the present claimed invention) were prepared by combining varying concentrations of MEGA-9 surfactant with PEI (0.1 wt%) in deionized water at 25 °C. The composition of Comparative Example D was prepared to include PEI (0.1 wt%) without any MEGA-9 surfactant. Each composition was exposed to the *S. aureus* suspension for 120 seconds according to the procedure of the "Microbial Kill Assay' described above. The results for Reference Examples 22-27, Comparative Example D, and the PBS Control Solution are reported in Table 7.

**Table 7.**

| | Percent MEGA-9 Surfactant relative to CMC | PEI Conc. (wt%) | Average Surviving Bacteria log(CFU)/mL | Bacteria Reduction log(CFU)/mL relative to Control |
|---|---|---|---|---|
| Ref-Example 22 | 10 | 0.1 | 7.36 | 1.24 |
| Ref-Example 23 | 50 | 0.1 | 7.11 | 1.49 |
| Ref-Example 24 | 100 | 0.1 | 6.21 | 2.39 |
| Ref-Example 25 | 125 | 0.1 | 5.81 | 2.79 |
| Ref-Example 26 | 150 | 0.1 | 5.49 | 3.11 |
| Ref-Example 27 | 200 | 0.1 | 5.21 | 3.39 |
| Comparative Example D | 0 | 0.1 | 7.47 | 1.13 |
| PBS Control | 0 | 0 | 8.60 | |

### Comparative Examples E1-E9

Compositions of Comparative Examples E1-E9 were prepared by adding varying concentrations of MEGA-9 surfactant to deionized water at 25 °C. Compositions E1-E9 did not contain any antimicrobial agent. Each composition was exposed to the *S. aureus* suspension for 120 seconds according to the procedure of the 'Microbial Kill Assay' described above. The results for Comparative Examples E1-E9 and Comparative Example A are reported in Table 8.

**Table 8.**

| | Percent MEGA-9 Surfactant relative to CMC | CHG Conc. (wt%) | Average Surviving Bacteria log(CFU)/mL |
|---|---|---|---|
| Comparative Example E1 | 10 | 0 | 9.08 |
| Comparative Example E2 | 25 | 0 | 9.14 |
| Comparative Example E3 | 75 | 0 | 9.12 |
| Comparative Example E4 | 90 | 0 | 8.96 |
| Comparative Example E5 | 100 | 0 | 9.08 |
| Comparative Example E6 | 110 | 0 | 9.12 |
| Comparative Example E7 | 125 | 0 | 9.12 |
| Comparative Example E8 | 175 | 0 | 9.10 |
| Comparative Example E9 | 190 | 0 | 9.01 |
| Comparative Example A | 0 | 0. 1 | 8.03 |

### Comparative Examples F1-F10

Compositions of Comparative Examples F1-F10 were prepared by combining varying concentrations of TERGITOL 15-S-7 surfactant (comparative surfactant) with CHG (0.1 wt%) in deionized water at 25 °C. Each composition was exposed to the *S. aureus* suspension for 120 seconds according to the procedure of the 'Microbial Kill Assay' described above. The results for Comparative Examples F1-F10 and the PBS Control Solution are reported in Table 9.

**Table 9.**

| | Percent TERGITOL 15-S-7 Surfactant relative to CMC | CHG Conc. (wt%) | Average Surviving Bacteria log(CFU)/mL | Bacteria Reduction log(CFU)/mL relative to Control |
|---|---|---|---|---|
| Comparative Example F1 | 10 | 0. 1 | 5.07 | 3.53 |
| Comparative Example F2 | 25 | 0. 1 | 4.46 | 4.14 |
| Comparative Example F3 | 75 | 0.1 | 6.14 | 2.46 |
| Comparative Example F4 | 90 | 0.1 | 5.75 | 2.85 |
| Comparative Example F5 | 100 | 0.1 | 5.04 | 3.56 |
| Comparative Example F6 | 110 | 0.1 | 7.49 | 1.11 |
| Comparative Example F7 | 125 | 0.1 | 5.74 | 2.86 |
| Comparative Example F8 | 170 | 0.1 | 7.78 | 0.82 |
| Comparative Example F9 | 190 | 0.1 | 7.65 | 0.95 |
| Comparative Example F10 | 0 | 0. 1 | 8.25 | 0.35 |
| PBS Control | 0 | 0 | 8.60 | |

### Comparative Examples G1-G4

Compositions of Comparative Examples G1-G4 were prepared by combining varying concentrations of TERGITOL 15-S-7 surfactant (comparative surfactant) with polyhexamethylene biguanide hydrochloride (PHMB) (0.01 wt%) in deionized water at 25 °C. Each composition was exposed to the *S. aureus* suspension for 30 seconds according to the procedure of the 'Microbial Kill Assay' described above. The results for Comparative Examples G1-G4 and the PBS Control Solution are reported in Table 10.

**Table 10.**

| | Percent TERGITOL 15-S-7 Surfactant relative to CMC | PHMB Conc. (wt%) | Average Surviving Bacteria log(CFU)/mL | Bacteria Reduction log(CFU)/mL relative to Control |
|---|---|---|---|---|
| Comparative Example G1 | 10 | 0.01 | 5.23 | 2.84 |
| Comparative Example G2 | 50 | 0.01 | 6.51 | 1.55 |
| Comparative Example G3 | 200 | 0.01 | 6.96 | 1.10 |
| Comparative Example G4 | 0 | 0.01 | 6.85 | 1.21 |
| PBS Control | 0 | 0 | 8.06 | |

### Comparative Examples H1-H6

Compositions of Comparative Examples H1-H6 were prepared by combining varying concentrations of MEGA-9 surfactant with octenidine hydrochloride (0.01 wt%) (comparative antimicrobial) in deionized water at 25 °C. Each composition was exposed to the *S. aureus* suspension for 30 seconds according to the procedure of the "Microbial Kill Assay' described above. The results for Comparative Examples H1-H6 and the PBS Control Solution are reported in Table 11.

**Table 11.**

| | Percent MEGA-9 Surfactant relative to CMC | Octenidine Conc. (wt%) | Average Surviving Bacteria log(CFU)/mL | Bacteria Reduction log(CFU)/mL relative to Control |
|---|---|---|---|---|
| Comparative Example H1 | 50 | 0.01 | 5.15 | 2.64 |
| Comparative Example H2 | 100 | 0.01 | 4.66 | 3.13 |
| Comparative Example H3 | 125 | 0.01 | 4.51 | 3.28 |
| Comparative Example H4 | 150 | 0.01 | 5.28 | 2.51 |
| Comparative Example H5 | 200 | 0.01 | 5.82 | 1.97 |
| Comparative Example H6 | 0 | 0.01 | 3.39 | 4.40 |
| PBS Control | 0 | 0 | 7.79 | |

### Comparative Examples I1-I7

Compositions of Comparative Examples I1-I7 were prepared by combining varying concentrations of MEGA-9 surfactant with CPC (0.1 wt%) (comparative antimicrobial) in deionized water at 25 °C. Each composition was exposed to the *S. aureus* suspension for 30 seconds according to the procedure of the "Microbial Kill Assay' described above. The results for Comparative Examples I1-I7 and the PBS Control Solution are reported in Table 12.

**Table 12.**

| | Percent MEGA-9 Surfactant relative to CMC | CPC Conc. (wt%) | Average Surviving Bacteria log(CFU)/mL | Bacteria Reduction log(CFU)/mL relative to Control |
|---|---|---|---|---|
| Comparative Example I1 | 10 | 0.1 | <3** | >6 |
| Comparative Example I2 | 25 | 0.1 | <3** | >6 |
| Comparative Example I3 | 75 | 0.1 | <3** | >6 |
| Comparative Example I4 | 100 | 0.1 | <3** | >6 |
| Comparative Example I5 | 175 | 0.1 | 4.57 | 4.61 |
| Comparative Example I6 | 190 | 0.1 | 6.42 | 2.76 |
| Comparative Example I7 | 0 | 0.1 | 8.20 | 0.98 |
| PBS Control | 0 | 0 | 9.18 | |

| | | | | |
|---|---|---|---|---|
| ** <3 indicates surviving bacteria count is below the limit of detection | | | | |

Illustrative embodiments of this invention are discussed and reference has been made to possible variations within the scope of this invention. It should be understood that this invention is not limited to the illustrative embodiments set forth herein. Accordingly, the invention is to be limited only by the claims provided below.

## Claims

1. A composition, comprising:
octanoyl-N-methylglucamide (MEGA-8);
wherein a concentration of octanoyl-N-methylglucamide (MEGA-8) is more than 170% of its critical micelle concentration in water at 25°C; and
an antimicrobial agent selected from the group consisting of a biguanide antimicrobial agent and a polymeric, cationic, non-micelle forming antimicrobial material;
wherein a concentration of the antimicrobial agent is no more than 5 wt%.

2. The composition of claim 1, wherein the concentration of octanoyl-N-methylglucamide (MEGA-8) is more than 180% of its critical micelle concentration in water at 25°C.

3. The composition of claim 1, wherein the concentration of octanoyl-N-methylglucamide (MEGA-8) is more than 190% of its critical micelle concentration in water at 25°C.

4. The composition of claim 1, wherein the concentration of octanoyl-N-methylglucamide (MEGA-8) is more than 200% of its critical micelle concentration in water at 25°C.

5. A composition, comprising:
octanoyl-N-methylglucamide (MEGA-8);
wherein a concentration of octanoyl-N-methylglucamide (MEGA-8) is more than 3.2 wt%; and
an antimicrobial agent selected from the group consisting of a biguanide antimicrobial agent and a polymeric, cationic, non-micelle forming antimicrobial material;
wherein a concentration of the antimicrobial agent is no more than 5 wt%.

6. The composition of claim 5, wherein the concentration of octanoyl-N-methylglucamide (MEGA-8) is more than 3.4 wt%.

7. The composition of claim 5, wherein the concentration of the octanoyl-N-methylglucamide (MEGA-8) is more than 3.5 wt%.

8. The composition of any one of claims 1-7, wherein the concentration of the antimicrobial agent is no more than 4 wt%.

9. The composition of any one of claims 1-8, wherein the concentration of the antimicrobial agent is no more than 3 wt%.

10. The composition of any one of claims 1-9, further comprising a glucamine compound.

11. The composition of claim 10, wherein the glucamine compound is selected from the group of D-glucamine, N-methyl-D-glucamine, N-Ethyl-D-Glucamine, N-Propyl-Glucamine, N-Butyl-D-Glucamine, N-octyl-D-glucamine, and combinations thereof.

12. The composition of any one of claims 1-11, further comprising a glucosamine compound.

13. The composition of claim 12, wherein the glucosamine compound is selected from the group of D-Glucosamine Hydrochloride, D-Glucosamine Sulfate, D-Glucosamine Phosphate, D-Glucosamine gluconate and combinations thereof.

14. The composition of any one of claims 1-13, further comprising a pharmaceutically acceptable carrier or solvent selected from the group consisting of water, alcohol, glycerol, polyethylene glycol, triethanolamine, methoxypropanol, isopropanol, ethyl acetate, polypropylene glycol and a combination thereof.

## Patentansprüche

1. Eine Zusammensetzung, umfassend:
Octanoyl-N-methylglucamid (MEGA-8);
wobei eine Konzentration von Octanoyl-N-methylglucamid (MEGA-8) mehr als 170 % seiner kritischen Mizellkonzentration in Wasser bei 25 °C beträgt; und
ein antimikrobielles Mittel, ausgewählt aus der Gruppe, bestehend aus einem Biguanid-antimikrobiellen Mittel und einem polymeren, kationischen, nicht mizellausbildenden antimikrobiellen Material;
wobei eine Konzentration des antimikrobiellen Mittels nicht mehr als 5 Gew.-% beträgt.

2. Die Zusammensetzung nach Anspruch 1, wobei die Konzentration von Octanoyl-N-methylglucamid (MEGA-8) mehr als 180 % seiner kritischen Mizellkonzentration in Wasser bei 25 °C beträgt.

3. Die Zusammensetzung nach Anspruch 1, wobei die Konzentration von Octanoyl-N-methylglucamid (MEGA-8) mehr als 190 % seiner kritischen Mizellkonzentration in Wasser bei 25 °C beträgt.

4. Die Zusammensetzung nach Anspruch 1, wobei die Konzentration von Octanoyl-N-methylglucamid (MEGA-8) mehr als 200 % seiner kritischen Mizellkonzentration in Wasser bei 25 °C beträgt.

5. Eine Zusammensetzung, umfassend:
Octanoyl-N-methylglucamid (MEGA-8);
wobei eine Konzentration von Octanoyl-N-methylglucamid (MEGA-8) mehr als 3,2 Gew.-% beträgt; und
ein antimikrobielles Mittel, ausgewählt aus der Gruppe, bestehend aus einem Biguanid-antimikrobiellen Mittel und einem polymeren, kationischen, nicht mizellausbildenden antimikrobiellen Material;
wobei eine Konzentration des antimikrobiellen Mittels nicht mehr als 5 Gew.-% beträgt.

6. Die Zusammensetzung nach Anspruch 5, wobei die Konzentration von Octanoyl-N-methylglucamid (MEGA-8) mehr als 3,4 Gew.-% beträgt.

7. Die Zusammensetzung nach Anspruch 5, wobei die Konzentration des Octanoyl-N-methylglucamids (MEGA-8) mehr als 3,5 Gew.-% beträgt.

8. Die Zusammensetzung nach einem der Ansprüche 1 bis 7, wobei die Konzentration des antimikrobiellen Mittels nicht mehr als 4 Gew.-% beträgt.

9. Die Zusammensetzung nach einem der Ansprüche 1 bis 8, wobei die Konzentration des antimikrobiellen Mittels nicht mehr als 3 Gew.-% beträgt.

10. Die Zusammensetzung nach einem der Ansprüche 1 bis 9, ferner umfassend eine Glucaminverbindung.

11. Die Zusammensetzung nach Anspruch 10, wobei die Glucaminverbindung ausgewählt ist aus der Gruppe von D-Glucamin, N-Methyl-D-glucamin, N-Ethyl-D-glucamin, N-Propyl-glucamin, N-Butyl-D-glucamin, N-Octyl-D-glucamin und Kombinationen davon.

12. Die Zusammensetzung nach einem der Ansprüche 1 bis 11, ferner umfassend eine Glucosaminverbindung.

13. Die Zusammensetzung nach Anspruch 12, wobei die Glucosaminverbindung ausgewählt ist aus der Gruppe von D-Glucosaminhydrochlorid, D-Glucosamin-sulfat, D-Glucosamin-phosphat, D-Glucosamingluconat und Kombinationen davon.

14. Die Zusammensetzung nach einem der Ansprüche 1 bis 13, ferner umfassend einen pharmazeutisch verträglichen Träger oder ein Lösungsmittel, ausgewählt aus der Gruppe, bestehend aus Wasser, Alkohol, Glycerol, Polyethylenglycol, Triethanolamin, Methoxypropanol, Isopropanol, Ethylacetat, Polypropylenglycol und einer Kombination davon.

## Revendications

1. Composition comprenant :
octanoyl-N-méthylglucamide (MEGA-8) ;
dans laquelle une concentration d'octanoyl-N-méthylglucamide (MEGA-8) est supérieure à 170 % de sa concentration critique de micelles dans l'eau à 25 °C ; et
un agent antimicrobien choisi dans le groupe constitué d'un agent antimicrobien biguanide et d'un matériau antimicrobien polymère, cationique, non micellaire ;
dans laquelle une concentration de l'agent antimicrobien n'est pas supérieure à 5 % en poids.

2. Composition selon la revendication 1, dans laquelle la concentration d'octanoyl-N-méthylglucamide (MEGA-8) est supérieure à 180 % de sa concentration critique de micelles dans l'eau à 25 °C.

3. Composition selon la revendication 1, dans laquelle la concentration d'octanoyl-N-méthylglucamide (MEGA-8) est supérieure à 190 % de sa concentration critique de micelles dans l'eau à 25 °C.

4. Composition selon la revendication 1, dans laquelle la concentration d'octanoyl-N-méthylglucamide (MEGA-8) est supérieure à 200 % de sa concentration critique de micelles dans l'eau à 25 °C.

5. Composition comprenant :
octanoyl-N-méthylglucamide (MEGA-8) ;
dans laquelle une concentration d'octanoyl-N-méthylglucamide (MEGA-8) est supérieure à 3,2 % en poids ; et
un agent antimicrobien choisi dans le groupe constitué d'un agent antimicrobien biguanide et d'un matériau antimicrobien polymère, cationique, non micellaire ;
dans laquelle une concentration de l'agent antimicrobien n'est pas supérieure à 5 % en poids.

6. Composition selon la revendication 5, dans laquelle la concentration d'octanoyl-N-méthylglucamide (MEGA-8) est supérieure à 3,4 % en poids.

7. Composition selon la revendication 5, dans laquelle la concentration de l'octanoyl-N-méthylglucamide (MEGA-8) est supérieure à 3,5 % en poids.

8. Composition selon l'une quelconque des revendications 1 à 7, dans laquelle la concentration de l'agent antimicrobien n'est pas supérieure à 4 % en poids.

9. Composition selon l'une quelconque des revendications 1 à 8, dans laquelle la concentration de l'agent antimicrobien n'est pas supérieure à 3 % en poids.

10. Composition selon l'une quelconque des revendications 1 à 9, comprenant en outre un composé de glucamine.

11. Composition selon la revendication 10, dans laquelle le composé de glucamine est choisi dans le groupe de D-glucamine, N-méthyl-D-glucamine, N-éthyl-D-glucamine, N-propyl-glucamine, N-butyl-D-glucamine, N-butyl-D-glucamine, et des combinaisons de ceux-ci.

12. Composition selon l'une quelconque des revendications 1 à 11, comprenant en outre une un composé de glucamine.

13. Composition selon la revendication 12, dans laquelle le composé de glucosamine est choisi dans le groupe de chlorhydrate de D-glucosamine, sulfate de D-glucosamine, phosphate de D-glucosamine, gluconate de D-glucosamine et des combinaisons de ceux-ci.

14. Composition selon l'une quelconque des revendications 1 à 13, comprenant en outre un support ou un solvant pharmaceutiquement acceptable choisi dans le groupe constitué d'eau, d'alcool, de glycérol, de polyéthylène glycol, de triéthanolamine, de méthoxypropanol, d'isopropanol, d'acétate d'éthyle, de polypropylène glycol et une combinaison de ceux-ci.
